# EUROPEAN PATENT SPECIFICATION

(11) **EP 3 700 614 B1**
(45) Date of publication and mention of the grant of the patent: **16.10.2024**
(21) Application number: 17808142.8
(22) Date of filing: 24.10.2017
(51) Int. Cl.: A61M 25/01, A61M 25/09

(54) **STEERABLE DEVICE AND SYSTEM**
LENKBARE VORRICHTUNG UND SYSTEM
DISPOSITIF ET SYSTÈME ORIENTABLE

(43) Date of publication of application: 02.09.2020
(73) Proprietor: Ecole Polytechnique Fédérale de Lausanne (EPFL), 1015 Lausanne (CH)
(72) Inventor: PETITPIERRE, Guillaume, 1255 Veyrier (CH); BOERS, Marc, 6064 Kerns (CH); RENAUD, Philippe, 1028 Preverenges (CH)
(74) Representative: Müller, Christoph Emanuel
(86) International application number: PCT/IB2017/056592
(87) International publication number: WO 2019/081962

(56) References cited:
- AU-B2- 2010 208 618
- US-A- 3 547 103
- US-A- 4 757 827
- US-A1- 2005 119 614
- US-A1- 2006 135 961

## Description

### Technical Field

The present invention belongs to the field of microtechnology. In particular, the invention features a guidewire for surgical operations.

### Background Art

Interventional radiology (IR) is a sub-speciality of radiology using minimally invasive image-guided procedures to diagnose and treat nearly all organs of the human body. The medical procedure mainly relies on both catheters allowing access through the vascular system (also includes the biliary tract, the gastrointestinal tract, etc.) and imaging methods (fluoroscopy, ultrasound, computed tomography) which allows precise navigation. IR is largely minimally invasive, as it takes advantage of the naturally present blood distribution system; for instance, vascular access is often provided through a single femoral artery/vein entry point, thus minimizing the risk to the patients and improving health outcomes. These procedures have been demonstrated to be less risky, to produce less pain and to decrease the recovery time in comparison to open surgeries.

Medical devices in IR are mainly composed of guidewires and catheters. At least the tip of these elements are radiopaque to enable image-guided navigation. The guidewires are introduced first in the vascular system and advanced in the blood vessels until reaching the targeted location. Catheters, which comprise a lumen, are advanced over guidewires which serve as a guiding element to reach the targeted location as well. Once the catheter is in place, it is used as a support channel for the introduction of other specific catheter devices aiming, for instance, at revascularization or embolization.

Guidewires have therefore the critical role of providing the initial access in order to reach a targeted region. Current guidewires can be either translated and/or rotated during this operation. They most often comprise a bent-shaped tip by which, through the external rotation and translation of the guidewire, the surgeon can select vascular intersections. Combined with imaging methods, this allows the surgeon to navigate the guidewire to the intended region.

The shape of the guidewire distal tip therefore largely impacts the navigation success of the device through the vascular system. Currently, guidewires are proposed with different pre-bended shapes, the surgeon selecting the best suited option according to the vessels geometry and the region he wants to reach. During the procedure, he commonly needs to replace the guidewire by another, more appropriate one. Similarly, the guidewire is often extracted from the patient, bended by hand by the surgeon (some guidewires propose this feature), before being re-introduced in the patient. This results in increased surgery time and procedure-related risks such as infections.

Guidewires replacement and distal tip repeated bending (by hand) is particularly frequent when the surgeon is confronted to tortuous blood vessels. This includes (and is not limited to) the neurovascular system, the cardiovascular system and the peripheral vascular system. In general, the blood vessels are more prone to present irregular, tortuous shapes at the distal portions of the vascular system (e.g. second and third level of the brain arteries).

Several solutions have been proposed in the past for solving the above shortcomings, in particular by providing steerable surgical tools to facilitate the surgeons' operation. For instance, US Patent Application 2016/0250449 describes a neuro-surgery assembly including a neuro-catheter slidably received over a wire guide. The wire guide includes an inner tube positioned within an elongated hollow tube that includes a pattern of openings at its distal segment to provide an atraumatic tip for reaching sensitive locations in the brain. The wire guide is changeable between a stiff condition in which the inner tube is pressurized and a soft condition in which the inner tube is depressurized. The user may switch between the stiff and soft conditions to negotiate the tortuous pathway to a brain treatment site without a need to swap out to a different wire guide to support advancement of the neuro-catheter.

WO 2015/164912 discloses an elongate, steerable apparatus such as a guidewire including an elongated body having at least one inner lumen, one, or more preferably multiple, tendons coupled to a distal bending region (e.g., the distal tip region) at a distal end and coupled to a proximal axial translation region at a proximal end. The proximal end may be configured to have multiple, in-line axial translation regions that each couple to a pull-wire or tendon so that axially moving the axial translation region relative to other regions of the device (e.g., pushing or pulling it longitudinally in the direction that the apparatus extends) may result in moving the pull-wire or tendon and bending the bendable distal region. The in-line axial translation regions may be connected to each other, e.g., elastically connected to each other via a spring or stretchable/compressible material.

In US 2014/0052109 it is described a steerable catheter probe having a body portion adapted for being connected to a proximal hub and a distal end portion connected to the body portion, wherein the catheter body defines a lumen; the distal end portion having a compressible segment and a non-compressible segment, the compressible segment having a longitudinal centerline; and a pull member attached to the distal end portion and adapted for applying a proximally directed force to the distal end portion whereby at least a portion of said compressible segment is compressed. Steering is provided by the positioning of the pull member and the design of the compressible segment: this comprises several compression coils, and at least one of its members of smaller diameter than the other larger and equal diameter coils. Pull wire is attached at fixation point to one of the large diameter coils distal to the small diameter coil, and within the lumen of large diameter coils but outside the lumen of any smaller diameter coils. Further examples of prior art steerable devices/guidewires are described for instance in US 5,203,772, US 2014/0343538, US 2006/0241519, US 2001/0037084, US 2008/0027285, US-A-2005/119614, AU-B-2010208618, US-A-4757827, US-A-2006/135961 and US 2016/0206853.

All the above-cited prior art solutions present several shortcomings. For instance, many of the known steerable guidewires/catheters are bulky, with a complicated actuation system and resulting in complex manufacturing, which raises production costs and limit the miniaturization of the final product, that in some instances is critical for e.g. navigating in tortuous, tiny blood vessels as found in the brain vascular network. Additionally, many of the known devices can deflect in several directions, a feature that enhance even more the fabrication complexity and the operation of the device itself, mainly due to sophisticated actuation means. In this context, it should be mentioned that medical practitioners intended to use guidewires or catheters are generally quite reticent to drift from common clinical practice; the use of such complex tools would require a certain training for mastering their use, and this could hinder or at least limit the adoption of the technology. Furthermore, actuators are always located at the very proximal end of the guidewire; up to the inventors' knowledge, no existing systems provide a clutchable manipulator which can be fixed by the surgeon at any desired portion of a guidewire's body while still providing actuation means to the deflectable tip.

There is therefore still need for simple, not expensive and user-friendly steerable devices such as guidewires or catethers, which can be reliably miniaturized to commonly used dimension in IR while providing enhanced control for navigating in tortuous blood vessel network.

### Summary of invention

Accordingly, the present inventors developed a guidewire according to claim 1.

In particular, the invention relates to a steerable device, embodied in some aspects of the invention as a guidewire for surgical procedures, and an associated system further comprising a handle with advanced features to allow a user to control the insertion and guiding of the device.

A first aim of the invention was to manufacture a steerable guidewire through a simple, reliable and cheap manufacturing process.

A second aim of the invention was to create a guidewire having steering properties which is easy to operate and manipulate so to access complex and tortuous vascular networks.

A further aim of the invention was to create a system comprising a steerable guidewire which can be used by an operator (such as a surgeon) by minimally drifting from the common clinical practice. All these aims have been accomplished by the present invention as defined hereinafter and in the appended claims.

A main consideration on which the invention is based is that devices for insertion into a subject's body do not need to bend or deflect in several axes, but on the contrary providing a single plane deflection would largely suffice in the context of interventional radiology, and could be actually preferred. As briefly summarized above, in clinical practice, a surgeon selects the size and the shape of guidewires depending on the needs and circumstances, and usually bends or pre-bends the distal, flexible end of the wire on one side in order to facilitate access thereof into tortuous paths. During this path finding procedure, performed with the help of imaging means, the user moves on the wire along a bodily cavity (such as a vessel) and twists it, often using a torquer device, according to the anatomy of said cavity in order to rotate the one-sided bent tip of the guidewire towards the selected vessel. Additionally, the imaging mean is adequately oriented perpendicular to the plane of the vessel intersection. As it can be understood, providing a single plane deflection guidewire, possibly actuated with user-friendly actuation means, would facilitate the directional positioning of the tip during operations while maintaining the common clinician's clinical practice, and avoiding the risks related to guidewire replacement or a tip re-shaping procedure.

It is therefore a first object of the present invention to provide for a steerable device having an elongated body defining a longitudinally-arranged lumen, said device comprising:
a) a proximal end portion adapted for handling by a user;
b) a distal end portion comprising a bendable portion and a tip; and
c) a pull wire connected to the distal end of the bendable portion and extending therefrom up to said proximal end along said lumen
characterized in that the bendable portion comprises a reinforcement structure positioned on one lateral side and a flexible, stress relief portion positioned on the opposed lateral side.

The bendable portion is located at the very distal end of the device, and the pull wire is connected to the tip.

The proximal end portion and/or the elongated body comprise an actuation region adapted to impart a force on the structure resulting in a distally-directed steering action on the bendable region. The actuation region is adapted to impart a tension force on the pull wire or an extension force on the elongated body resulting in a distally-directed force to the bendable region. In particular, the actuation region is adapted to impart a proximally-directed force on the pull wire or a distally-directed force to the bendable region.

In one embodiment, said actuation region comprises a coiled member.

In one embodiment, the reinforcement structure is integral part of the elongated body.

In one embodiment, the bendable portion is compressible.

In one embodiment, the bendable portion comprises a coiled member.

In one embodiment, the flexible, stress relief portion comprises a plurality of cut-outs located on one lateral side of the bendable portion.

In one embodiment, the device is configured as a guidewire for insertion into a subject's body.

Another aspect of the present invention relates to a system comprising the above-described device and an actuator adapted to impart force on the structure resulting in a distally-directed steering action on the bendable region, such as a tension force on the pull wire resulting in a distally-directed force to the bendable region. In particular, the actuator can impart a proximally-directed force on the pull wire or a distally-directed force on the bendable portion.

In one embodiment, the system is characterized in that the actuator is a torque device comprising:
a) a first handle comprising:
   - an elongated body, a proximal end and a distal end, said handle defining a lumen along its entire length, and said distal end comprising means for gripping and ungripping the above-described steerable device;
b) a second handle comprising:
   - an elongated body, a proximal end and a distal end, said handle defining a lumen along its entire length, and said distal end comprising means for gripping and ungripping the above-described steerable device;
wherein the first and second handles are arranged in a coaxial configuration so that they can perform a longitudinal relative displacement.

In one embodiment, said gripping and ungripping means comprise a spring collet clamp.

In one embodiment, said distal ends of said first and second handles each comprise a tapered tip adapted to engage said gripping and ungripping means and tighten them upon a longitudinal relative displacement of said first and second handles.

In one embodiment, the first and second handles are connected through an accordion-like, corrugated element.

In one embodiment, the first and second handles are connected through a spring clip.

In one embodiment, the first and second handles are arranged in a coaxial male-female configuration.

In one embodiment, each of said first and second handles comprises a plurality of corrugations on their inner lumen adapted to work as snaps.

In one embodiment, the system further comprises a first and second tapered fasteners adapted to releasably engage and tighten said gripping and ungripping means of said first and second handles, respectively.

In one embodiment, one of the first or second handle comprises a longitudinal slot along its body, and the other of the first or second handle comprises a protrusion adapted to fit and slide along said longitudinal slot.

In one embodiment, the system further comprises a gear rotational actuator adapted to engage said protrusion and operate upon rotation a longitudinal relative displacement of said first and second handles.

### Brief description of drawings

In the Figures:
Figure 1 depicts one embodiment of the device of the invention: A) overview of the device; B) lateral view; C) bottom view; D) longitudinal cross-section;
Figure 2 depicts two alternative embodiment of the device of the invention: A) lateral and longitudinal cross-section of the device having the inner pull wire connected to the distal end of the bendable portion; B) lateral and longitudinal cross-section of the device having the inner pull wire connected to the tip, wherein the bendable portion is located proximally compared to said tip;
Figure 3 depicts two lateral views of the device in a rest and actuated positions; notice the compression of the stress relief elements (coils) on the inner side of the bend, and the constriction of the coils on the outer side of the bend;
Figure 4 shows for alternative embodiments of the reinforcement structure: A) rectangular; B) triangular; C) rhomboidal; D) "butterfly" shape;
Figure 5 shows an alternative embodiment of the device of the invention comprising a segmented reinforcement structure: A) lateral view and B) bottom view of the device in a rest position; C) lateral view of the device in an actuated position, showing elbow bends;
Figure 6 shows a longitudinal cross section of an alternative embodiment of the device of the invention, characterized in that at least the bendable portion is composed of a solid body made for instance of a metal or a soft, polymeric material, said body having a plurality of cutouts defining the stress relief portion;
Figure 7 shows alternative embodiments of the device of Figure 6, in which the cutouts have a triangular (A), rounded (B) or rectangular (C) appearance;
Figure 8 shows two longitudinal cross sectional views of the actuation portion in a rest and actuated positions;
Figure 9 depicts an overview of one embodiment of a system according to the invention, comprising the steerable device and an actuator;
Figure 10 shows one embodiment of the actuator, having two handles connected via an accordion-like element;
Figure 11 shows two embodiments of the actuator, having two handles connected via A) a spring and a displacement element or B) a spring tab or clip;
Figure 12 shows one embodiment of the actuator: A) exploded view and B) isometric view;
Figure 13 shows a longitudinal cross sectional view of the actuator of Figure 12, as well as of the fasteners;
Figure 14 shows a partial longitudinal cross sectional view of one embodiment of the actuator comprising two handles interacting by means of snaps;
Figure 15 shows one embodiment of the actuator comprising a gear rotational element: A) exploded view and B) isometric view;
Figure 16 shows a longitudinal cross sectional view of the actuator of Figure 15 both in a rest position (A) or in operation (B);
Figure 17 shows a comparison between state-of-the-art, conventional pre-bent guidewires (A) and the device of the invention (B); notice the ability of the steerable guidewire to reach sub-arteries not reachable with classic guidewires;
Figure 18 depicts the actuator-driven actuation of the device of the invention, in which a distally-directed force imparted on a coiled actuation region of the device allows deflection thereof.

### Description of embodiments

The present disclosure may be more readily understood by reference to the following detailed description presented in connection with the accompanying figures, which form a part of this disclosure. It is to be understood that this disclosure is not limited to the specific conditions or parameters described and/or shown herein, and that the terminology used herein is for the purpose of describing particular embodiments by way of example only and is not intended to be limiting of the claimed disclosure.

As used herein and in the appended claims, the singular forms "a", "an" and "the" include plural referents unless the context clearly dictates otherwise. Also, the use of "or" means "and/or" unless stated otherwise. Similarly, "comprise", "comprises", "comprising", "include", "includes" and "including" are interchangeable and not intended to be limiting. It is to be further understood that where descriptions of various embodiments use the term "comprising", those skilled in the art would understand that in some specific instances, an embodiment can be alternatively described using language "consisting essentially of" or "consisting of."

The present invention is directed to a steerable device capable of bending, deflecting, or otherwise being controlled off-axis, which is some instances is embodied as a guidewire adapted to move within a certain target location such as a vessel, body tissue or otherwise hollow organ.

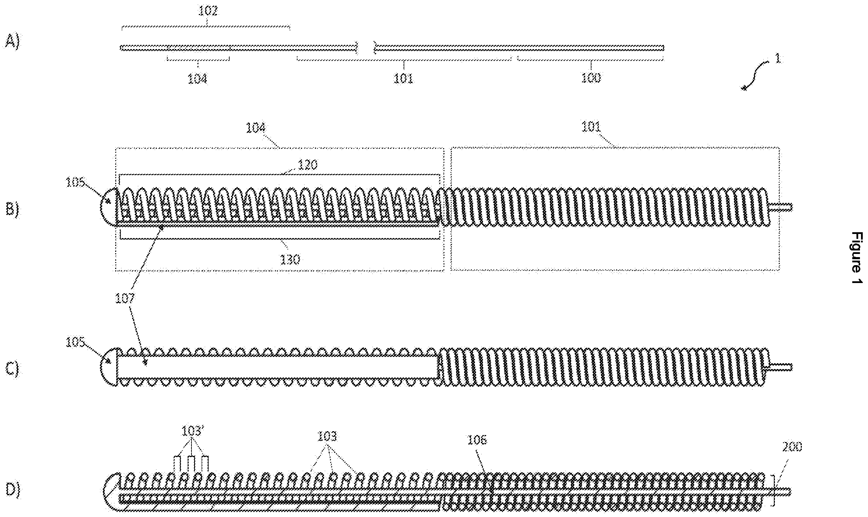

With reference to Figure 1, one embodiment of the device of the invention is shown. Device 1 comprises a proximal end 100, an elongated body 101 and a distal end portion 102. A lumen 200 spans from the distal end 102 up to the proximal end 100 and along the entire body 101, said lumen being coaxially arranged compared to the longitudinal axis of the device. The distal end portion 102 comprises a bendable portion 104 and a tip 105 at its very end. Tip 105 is generally an atraumatic and non-sharp tip, with a rounded, oval, or similar appearance. Materials opaque to X-rays, such as platinum, gold, tungsten, tantalum or the like, may be advantageously incorporated therein, to act as a fluoroscopic marker to aid in visualization. The bendable portion 104 is a region at the distal end 102 of the device that is significantly more flexible and susceptible to deflection than the remaining body 101 and/or proximal region 100.

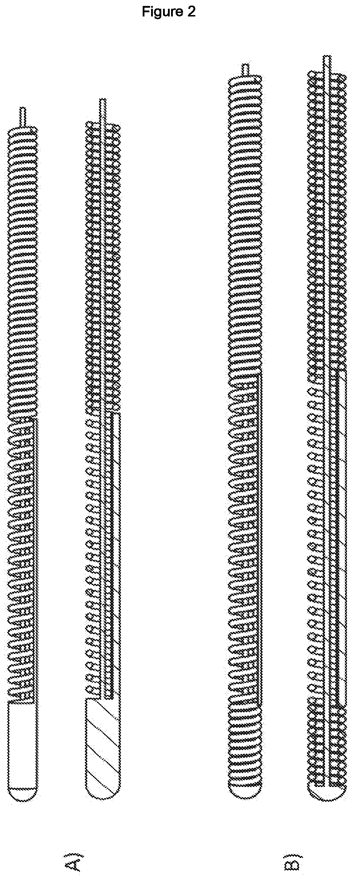

A pull wire 106 is affixed to the distal end of the bendable portion 104 and extends therefrom up to said proximal end 100 along said lumen 200. In the depicted embodiment, the bendable portion 104 is located at the very distal end of the device, and the pull wire 106 is connected to the tip 105; alternative embodiments are shown on Figure 2. The device is characterized by the fact of comprising, in the bendable portion 104, a reinforcement structure **107** advantageously located on one lateral side and a flexible, stress relief portion **120,** located on the opposed lateral side.

The steerability, deflection or bending of a distal region **102** of the guidewire device, in particular of the bendable portion **104** thereof, is obtained by imparting a longitudinal tension force on the pull wire **106.** For instance, this can be accomplished by using the inner pull wire **106,** longitudinally arranged with respect to the device's body inside the lumen **200** thereof, and free to translate along the device longitudinal axis. Said pull wire **106** is axially rigid while being flexible, and a translational motion of said wire **106** relatively to the lumen **200** along the longitudinal axis, through application of a tensile strength in a proximal direction, is used to generate the deflection. In another scenario, however, the deflection of the bendable portion **104** can be obtained by applying a distally-directed force on it when the pull wire **106** is further affixed at the proximal end **100,** as it will be detailed later on.

The steerable device is fabricated so that it is substantially straight from its proximal end **100** to its distal end **102.** As will be explained later on in more details, manipulation of a control mechanism at the proximal end **100** and/or body **101** causes the distal, bendable region **104** of the steerable device to bend or curve away from its longitudinal axis.

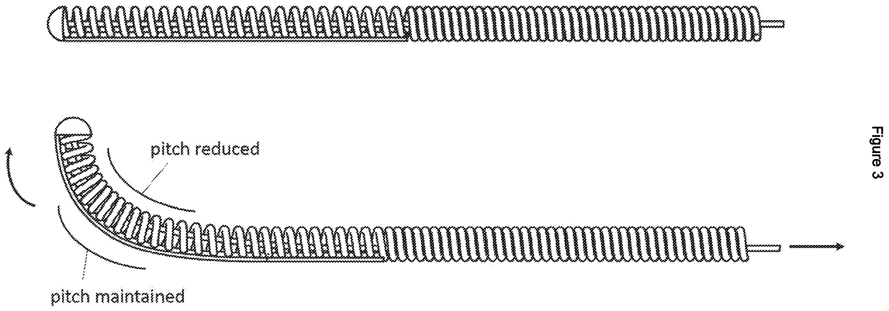

The bendable region **104** is characterized by the presence of two distinct portions, each positioned on one side of said region **104,** and one opposed to the other, that are named herein for the sake of clarity and simplicity stress relief portion **120** and constrained portion **130.** Those two portions are designed to induce an asymmetric compression in the bendable region **104** that guides the direction of bending when an axial force (stress) is applied thereon by the pull wire **106.** To do so, the stress relief portion **120** comprises a series of stress relief elements **103** separated by hollow spaces **103'** that allows for the compressibility of the bendable region **104;** on the contrary, the constrained portion **130** is characterized by the presence of a so-called reinforcement structure **107** that limits the compressibility of one side of said bendable region **104** so that the generation of a compression stress will induce a bending which is opposed to the side of the reinforcement structure **107,** as schematically depicted in Figure 3. As it will be therefore apparent, the device of the invention is characterized by the fact of being bendable or otherwise deflectable on a single side only.

In one embodiment, the entire device or portions thereof can comprise or consist of a coiled member. In particular, the distal end portion **102,** particularly the bendable portion **104** of this latter, and the proximal end **100** and/or the device body **101** can comprise or be formed as a helix or a coil. In preferred embodiments, the bendable portion **104** can comprise or be formed as a helix or a coil having a finite spacing (**103**') between the coils (**103**) or windings. As a result of this configuration, the bendable portion **104** mechanically results as a loosely wound spring coil, so that adjacent windings or coils **103** are not in contact with one another in the absence of any tensile stress. As is typically the case for guidewires designed to navigate vessels of humans and animals, and used as a guide for e.g. placement of a catheter, the proximal end **100** and the body **101** are formed by a tightly wound coil of a high tensile strength wire of a resilient, noncorrosive metal such as stainless steel, nitinol, platinum or other biocompatible materials, as well as any combination of the foregoing. This arrangement stiffens the device's shaft except for the distal end, providing sufficient column strength so that it can advantageously be pushed, from the proximal end **100,** to force the distal portion **102** through the vasculature of a patient, while said portion **102** is flexible enough to be deflected by blood vessel wall so that it may impinge on the blood vessel walls during passage without deforming, puncturing or injuring the blood vessel. Thus, referring to Figure 3, it can be seen that a lateral deflection of steering region (i.e., the bendable portion **104**) to one side may involve an axial compression of adjacent wire loops (pitch) on the inside curve (stress relief portion **120**) of the bend, while constraining or otherwise limiting the pitch on the outside curve (constrained portion **130**) of the bend.

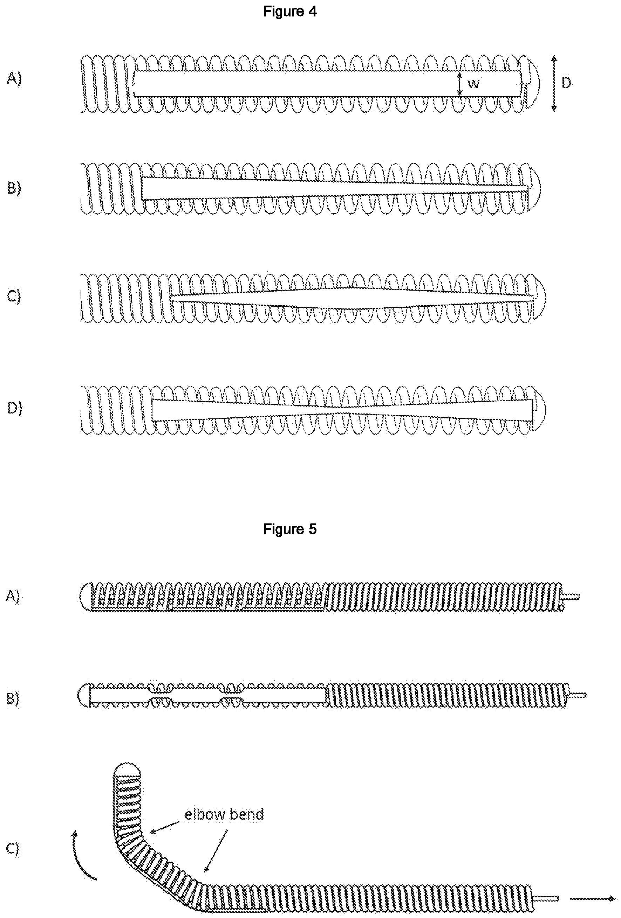

In some embodiments, the reinforcement structure **107** can have various different shapes, and even more than one reinforcement structures **107** can be present along the bendable portion **104.** Moreover, in some embodiments the reinforcement structure **107** can span from the distal tip **105** all along the bendable portion **104,** so that a constrained portion **130** would cover one entire side thereof, or the reinforcement structure **107** can define a more limited portion of bendable portion **104** as a constrained portion **130.** As depicted in the various embodiments in Figure 4, the reinforcement structure **107** may have e.g. a rectangular, triangular, rhomboidal or any other suitable contour appearance depending on the needs and circumstances; the contour shape would define the resistance to the bending of the constrained portion **130** upon application of a tensile stress, which will be higher in those portions of the constrained region **130** having larger width of the reinforcement structure **107** and vice versa, assuming an homogeneous thickness of the reinforcement structure. Moreover, as depicted e.g. in Figure 5, more than one reinforcement structure **107,** or a sectioned one, can be implemented on the bendable portion **104** in order to set a plurality of constrained portions **130.** This embodiment is particularly useful for obtaining "elbow" deflections of the bendable portion **104,** wherein by "elbow" deflection it is meant that the bend in the bendable portion **104** does not occur in a uniform way along the bendable portion **104** but at a relatively discrete position(s) displaced proximally from the tip **105** of the guidewire. This arrangement could enable or facilitate negotiation of the artery with e.g. minimal trauma to the vascular intima.

Typically, the bendable portion **104** is smaller or equal in length than the reinforcement structure **107** length, such as about from 5% to 105% of reinforcement structure **107** length. A typical range for the width of reinforcement structure **w** is 5% to 95% of the bendable portion **104** diameter, **D.** The reinforcement structure **107** profile more preferably follows the curvature of the coiled member structure when observed through an axial view facing a cross section. Moreover, a typical range for coil spacing in the bendable portion **104** is +5% to 1000% of the coil spacing in a coiled body region **101.**

In some embodiments, the reinforcement structure **107** is substantially made of an elastic and/or stretchable material such as for instance various elastomers, silicon polymeric materials like Polydimethylsiloxane (PDMS), silicon adhesives, silicone rubbers, natural rubbers, stretchable fabric, thermoplastic elastomers, hydrogels, polyamide, polyimide. In some additional or alternative embodiments, the reinforcement structure 107 is substantially made of non-elastic, stiff materials such as for instance poly ethylene (PE), poly propylene (PP), polyether etherketone (PEEK), Acrylonitrile butadiene styrene (ABS), epoxys, polytetrafluoroethylene (PTFE), polyurethane, thermoplastic polyurethanes (TPU), Nylon, Polyether block amide (PeBax), kevlar and liquid-crystal polymers or metals such as for instance stainless titanium, steel, nickel titanium alloy (Nitinol), tungsten, cobalt, chrome, nickel, aluminium, copper, molybdenum or any combination thereof. This embodiment is particularly suitable and advantageous in situations when an elbow deflection is sought, as shown in Figure 5.

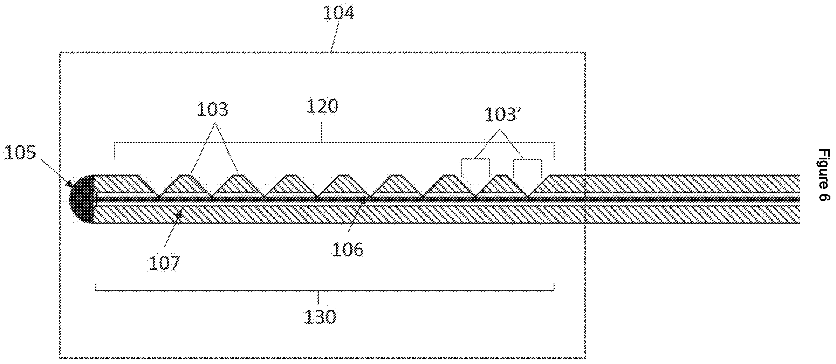

In another embodiment according to the invention depicted in Figure 6, the device is composed, at least at its distal end **102** and particularly at the bendable region **104,** of a solid body, such as a soft, polymeric body, containing a thru hole defining the lumen **200** in which a pull wire **106** is inserted. The pull wire **106** is attached by any suitable means such as for instance gluing on the distal end tip **105** of the solid body. The bendable region **104** of the solid body, which can have any lateral cross-section (perpendicular to the longitudinal axis) such as round, elliptic, square, rectangular and the like, comprises a plurality of stress relief elements **103** in its stress relief portion **120** embodied as cutouts. In embodiments where the elongated body **101** of the device is also made of the same material of said solid body, such as a polymeric material or a metal, the reinforcement structure **107** can constitute an integral part of said body **101.** Briefly, a tubular polymeric element can be shaped to include a plurality of cutouts on one side of one of its extremities while leaving the other side unaltered, so to define a built-in blocked lateral side **130** and a lateral bendable side **120.**

In the embodiment represented in Figure 6, the distal end **102** of a soft polymeric element is rendered single-sided deflectable by cutting slots or gaps **103'** generally having hemispherical, rounded, trapezoidal, square or triangular profile, those gaps **103'** not passing completely through the entire thickness of the polymeric element so that a sort of spine with ribs is formed.

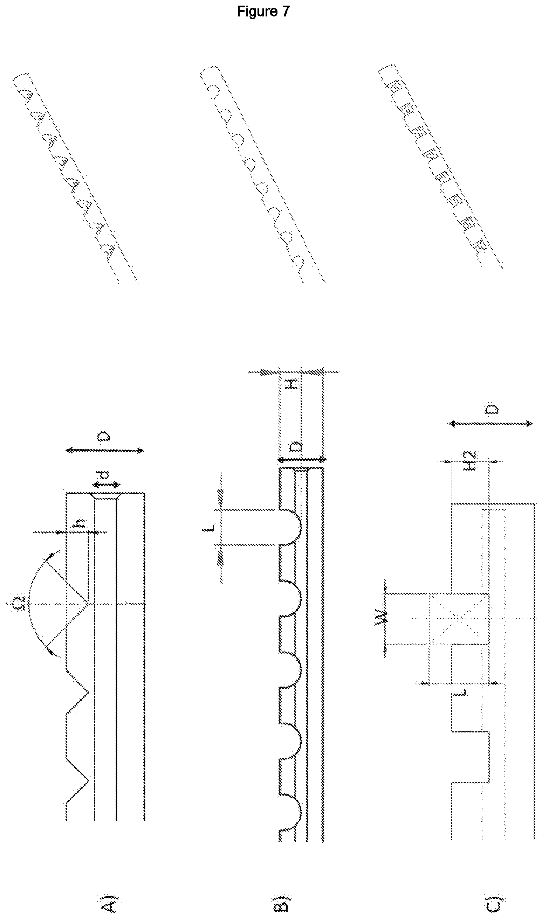

As better shown in Figure 7A depicting a triangular cutouts embodiment, the ratio between the guidewire inner diameter **d** and the guidewire outer diameter **D** is typically comprised between 1:10 and 1:1.1, the cutout angle **Ω** ranges from about 5° to 165°, typically from 30° to 120°, and the cutout height **h** ranges from about 5% to 95% of **D,** typically from 10% to 50% of **D.** In a rounded cutouts embodiment (Figure 7B), a typical cutout height **H** ranges from about 5% to 95% of **D** (starting from central axis), typically from 50% to 75% of **D,** with an **L** to **H** ratio comprised between 0.01 and 10. In a rectangular cutouts embodiment (Figure 7C), a typical ratio between the height of rectangular cutout **L** and the width of rectangular cutout **W** ranges from 1:1 to 1:8, and a typical cutout height **H2** ranges from about 5% to 95%, typically from 10 to 50% of **D,** with **L** ≥ **H2.** The number of cutouts typically ranges from 1 to 300, and the bendable region **104** can span e.g. from 1 mm up to 10 cm.

A steerable guidewire according to the invention can be fabricated to have diameters ranging from about 0.007 inches to about 0.038 inches (1 inch = 2.54cm) The external diameter of wire wound guidewires will of course be a function of the intended application. For instance, a steerable guidewire according to the invention intended for coronary angioplasty is preferably wound to have an external diameter in the range of from about 0.014" to about 0.018".

The device of the invention embodied as a guidewire can come in various stiffnesses, and its length can range from about 50-cm to about 350-cm or longer. Typical guidewire diameters for cardiovascular use have diameters in the range of 0.007" to 0.018" to 0.025", a length in the range of 100 to 300 cm, and a tip stiffness of 1 to 10 grams for access to unoccluded vessels, and 10 to 30 grams for crossing occluded or stenosed vessels. Tip stiffness is measured by the amount of force/weight needed to deflect the tip by 45%. Peripheral guidewires are larger in diameter (in the range of about 0.032", 0.038", or greater) and may have a higher tip stiffness. A key advantage of the inventive concept behind the invention relies on the positioning of a reinforcement structure **107** that minimally alter the entire structure of a guidewire, rendering even very thin wires adapted to deflect without the need of bulky apparatuses or actuation mechanisms.

For some applications, such as coronary access, the distal end **102** will be floppier (have a lower stiffness) than the body **101** of the guidewire, while in other applications, such as aortic access, the distal end **102** elongated body **101** may have substantially similar stiffness. The distal end **102** may extend 0.5 to 10 cm, such as 1 to 5 centimeters.

In some embodiments, the exterior surface of the device is entirely or partially equipped with an elastic, biocompatible coating or sheath to provide a smooth outer surface. Suitable coatings can be formed by dipping, spraying or wrapping and heat curing operations as are known in the art. A coating material is selected to minimize sliding friction of the device during insertion and removal into a subject's body, and is substantially chemically inert in the in vivo vascular environment. A variety of suitable materials are known, including, for example, polytetrafluoroethylene (PTFE), tetrafluoroethylene (TFE), urethane, polyurethane, thermoplastic polyurethanes (TPU), silicone Polyether block amide (PeBax), Nylon or polyethylene.

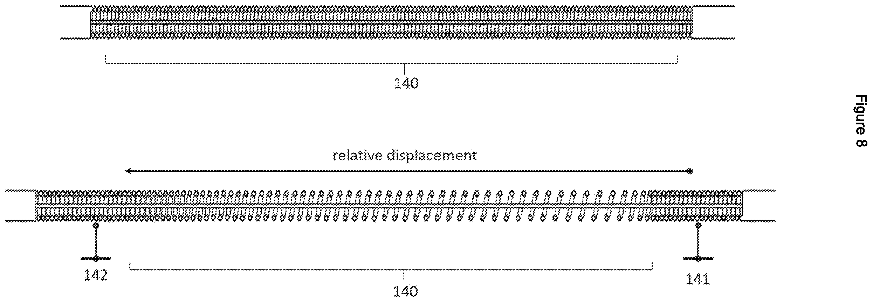

The steerable device or guidewire of the invention further comprises an actuation region **140** at its proximal end **100** and/or anywhere else along the body **101** of the device which is adapted to impart a tension force on the pull wire **106** resulting in a bending of the region **104,** such as a proximally-directed force on the pull wire **106** or a distally-directed force on the bendable region **104,** in order to control the articulation at the distal end **102,** particularly at the bendable region **104.** The actuation region **140** is designed to permit physical access to the pull wire **106** and manipulation thereof; for instance, the actuation region **140** can be placed at the very proximal end extremity of the device where the proximal end of the pull wire **106** can be directly clamped, grasped or otherwise manipulated to impart longitudinal forces thereon. Direct access to the pull wire **106** can however be attained anywhere along the proximal region **100** and/or the elongated body **101.** In preferred embodiments, the actuation region **140** is elastic in nature, and can comprise or consist of a coiled member, as depicted in Figure 8. In the shown scenario, the device is embodied as a guidewire having its body **101** formed by a tightly wound coil of a high tensile strength metal wire, a pull wire **106** affixed distally on the tip **105** and proximally to the proximal end **100,** and the steering mechanism of action consists of a collapse of the bendable region **104** by means of longitudinal stress forces applied on the actuation region **140.** In particular, the coiled portion of the actuation region **140** gets blocked, e.g. by clamping, on two opposed, longitudinally-placed anchor points **141** and **142.** The relative displacement of the blocking point **141** and **142** imparts an axially, longitudinally directed force on the entire body **101** up to the distal end **102,** which tends to collapse the hollow spaces **103'** in the stress relief portion **120** of the bendable region **104,** finally bringing the stress relief elements **103** closer together.

Especially in embodiments of the invented device where a guidewire is envisaged, the actuation mechanism can be favoured by the use of an actuator adapted to impart a proximally-directed force on the pull wire **106** or a distally-directed force on the bendable portion **104.**

This is mainly due to the physical and mechanical nature (e.g. a smooth surface), as well as the size (e.g. small diameter), of guidewires conventionally used in clinical practice, which renders their manipulation hard to be performed by hands. In this contexts, medical practitioners are used to operate guidewires with the help of small devices adapted to favour the grip and the torque of said wires, thus facilitating manoeuvrability thereof. A so-called torque device is used to provide a "handle" whereby the surgeon can have maximum control over the positioning and orientation of the guidewire.

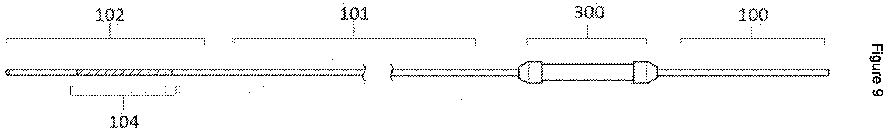

Accordingly, a further object of the present invention relates to a system comprising the device 1 of the invention and an actuator adapted to impart a proximally-directed force on the pull wire **106,** or a distally-directed force on the bendable portion **104.** The actuator, also referred to herein as "torque device", "torque actuator" or "torquer", features a controlling mechanism for permitting the articulation at the distal end **102** of the device of the invention, and can advantageously be releasably affixed to the proximal end **100** and/or body **101** of this latter to follow the advancement of the device along a path, such as the endoluminal space of a blood vessel, and allowing negotiation, pushing/pulling and steering operations (Figure 9).

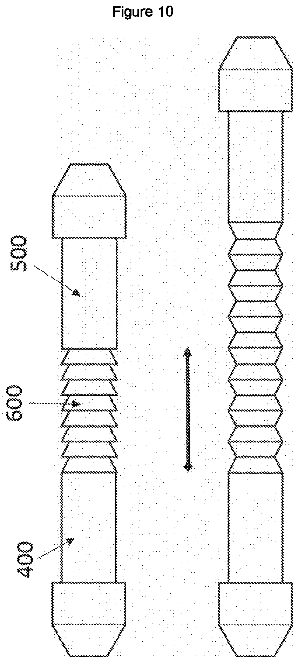

In the implementation of the invention, a plurality of non-limiting embodiments of said actuator **300** have been developed, depicted mainly in Figures 10 to 16. Generally speaking, the developed torque actuator 300 comprises:
a) a first handle **400** comprising:
   - an elongated body **401,** a proximal end **402** and a distal end **403,** said handle **400** defining a lumen **404** along its entire length, and said distal end **403** comprising means **410** for gripping and ungripping the above-described steerable device **1;**
b) a second handle **500** comprising:
   - an elongated body **501,** a proximal end **502** and a distal end **503,** said handle defining a lumen **504** along its entire length, and said distal end **503** comprising means **510** for gripping and ungripping the above-described steerable device **1;**
wherein the first and second handles **400** and **500** are arranged in a coaxial configuration so that they can perform a longitudinal relative displacement.

A torquer **300** according to the invention securely holds and controls a guidewire with one hand and with smooth operation. The actuator is simple in its construction, fast to load and adjust, easily gripped and ungripped and free of jerking wire movements during operating procedures.

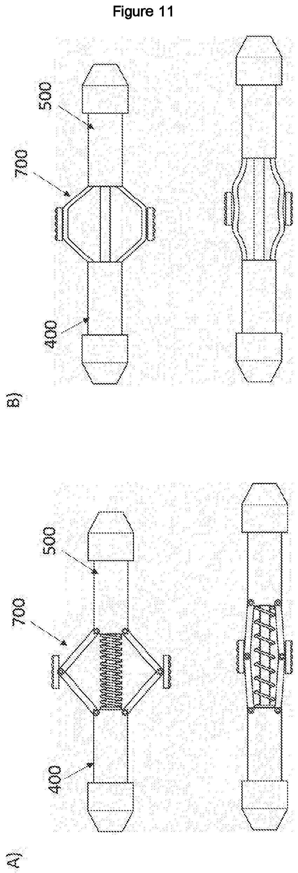

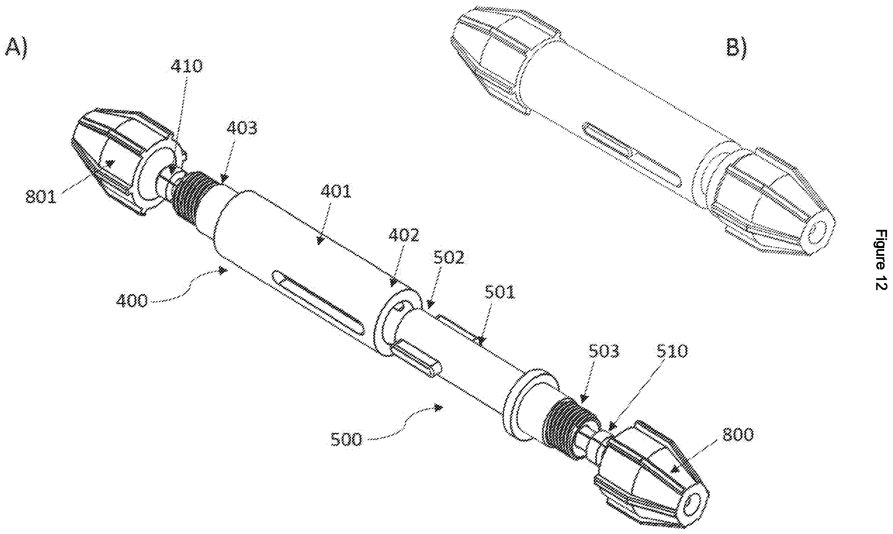

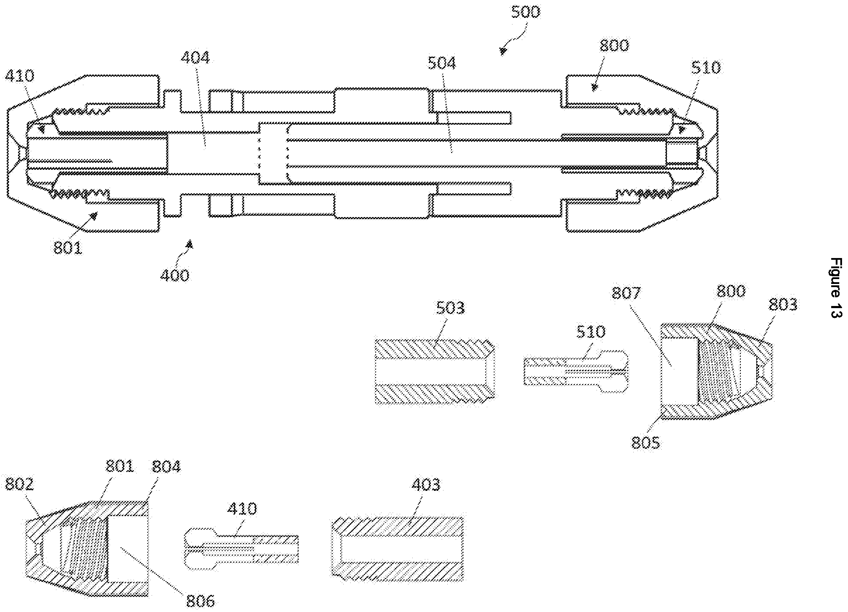

The torquer **300** is composed of two handles **400** and **500** connected or connectable between them through a variety of solutions; as a way of example, said handles **400** and **500** can be coupled and/or fixed one with respect to the other by means of an accordion-like, corrugated element **600** (Figure 10), via a spring clip **700** (Figure 11A and B), or thanks to a male-female arrangement, permitting to insert e.g. handle **400** into handle **500** (Figure 12A and B; Figure 13). Notwithstanding the connection setup, handles **400** and **500** are substantially coaxially arranged one with respect to the other along their longitudinal axes.

As exemplary depicted in Figure 12A and B and Figure 13, an interior long axial channel or lumen (**404, 504**) extends between the proximal end (**402, 502**) and the distal end (**403, 503**) through the elongated body (**401, 501**) of each of the first and second handles **400** and **500**. An aperture formed at the proximal end (**402, 502**) and an aperture formed in the distal end (**403, 503**) provide access to the passage, and facilitate advancement, of a guidewire **1** through body. Channels or lumens **404** and **504** are substantially coaxially arranged one with respect to the other along their longitudinal axes so to create, upon connection of handles **400** and **500**, a unitary passage or channel.

In preferred embodiments, gripping and ungripping means **410** and/or **510** comprise a spring collet clamp. A collet locking system comprises a chuck that forms a collar around an object to be held (in the present case, guidewire device 1) and exerts a strong clamping force on said object when it is tightened. The collet is a sleeve with an inner surface normally matching that of a guidewire and a conical outer surface. The collet can be squeezed against a matching taper such that its inner surface contracts to a slightly smaller diameter, squeezing the guidewire to hold it securely. This is achieved with a spring collet, made of e.g. spring steel, with one or more kerf cuts along its length defining at least two flanges that allow the spring collet to expand and contract. With enough compression, the collet locking mechanisms **410** and **510** exert sufficient frictional force upon the guidewire **1** such that this is "locked" relative to torque device **300,** preventing the guidewire from sliding freely within the hollow inner bores **404** and **504** of torque device **300.**

To perform said compression, in one embodiment the system further comprises a first and second tapered fasteners **800** and **801** adapted to releasably engage and tighten said gripping and ungripping means **410** and/or **510,** such as a spring collect clamp, of said first and second handles **400** and **500,** respectively. As shown for instance in Figures 12 and 13, fasteners **800** and **801** are caps including a first end (**802, 803**) and a second end (**804, 805**), with a hollow passage extending therebetween (**806, 807**). An aperture formed in the first end (**802, 803**) and an aperture formed in the second end (**804, 805**) provide access to the passage and facilitate advancement of a guidewire 1 through the cap (**800, 801**). Hollow passages **806** and **807** are substantially coaxially arranged with respect to channels or lumens **404** and **504** along their longitudinal axes so to create, upon connection with handles **400** and **500,** a unitary passage or channel. The first end (**802, 803**) is tapered so that, upon connection with distal end (**403, 503**) of a handle (**400, 500**), the spring collect clamp (**410, 510**) is locked against a wire inserted therein. Threads formed adjacent the second end (**804, 805**) on an interior of the fastener (**800, 801**) can cooperate with threads at the distal end (**403, 503**) of a handle (**400, 500**).

In one additional or alternative embodiment, said distal ends **403** and **503** of said first and second handles **400** and **500** each comprise a tapered tip adapted to engage said gripping and ungripping means **410** and/or **510** such as a spring collect clamp and tighten them upon a longitudinal relative displacement of said first and second handles **400** and **500**. In this embodiment, a collet clamp **410** or **510** is designed as a tubular element coaxially arranged along its longitudinal axis within the hollow elongated body of the handle (**401** or **501**). The diameter of the collet (**410**, **510**) matches the bore (**401**, **501**) of the handle (**400**, **500**), having the larger, distal tapered end slightly greater than the bore while the smaller, proximal diameter is slightly less than the bore. A longitudinal relative displacement of the first and second handles **400** and **500** pushes the distal ends **403** and **503** thereof against the distal, larger end of the collet **410** and **510**, forcing the tapered distal end of the collet clamp (**410**, **510**) to slide within the hollow elongated body of the handle (**401** or **501**), thus locking the two elements together. Upon release of the force necessary to perform the relative displacement of handles **400** and **500**, the elastic spring nature of the collet (**410**, **510**) allows this latter to be pushed outside the body (**401**,**501**), thus consequently releasing the clamping force. As it will be evident to a person skilled in the art, combinations of the above-described embodiments are also envisageable; for instance, in operation, the first handle **400** is proximally arranged compared to the operator, while the second handle **500** is distally located. The operator can fix a proximally-located anchorage point **141** by screwing the fastener **800** about the threaded distal end **403** of handle **400,** so to tight the collet **410** and apply a clamping force on the device **1.** In this way, the actuator **300** can be operated as a torquer conventionally used in clinical practice; however, the distal end **503** of distally-located handle **500** can be tapered as to engage the collet **510** and automatically clamp and tighten it on an anchorage point **142** upon a longitudinal relative displacement of said first and second handles **400** and **500.**

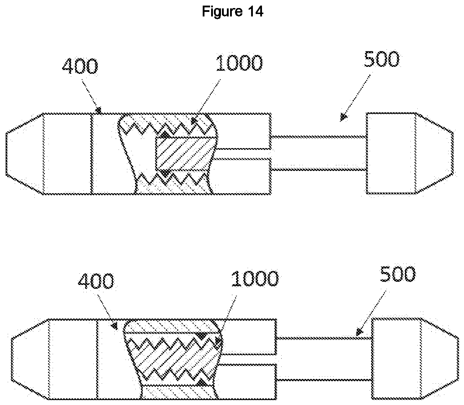

Moreover, in a further embodiment according to the invention, each of said first and second handles **400** and **500** can comprise a plurality of corrugations **1000** on their inner lumen adapted to work as snaps. In this way, handles **400** and **500** can be fixed one relative to the other in defined positions depending on the needs (Figure 14). This feature is particularly advantageous for providing a tactile feedback to the operator, and to facilitate the incremental actuation of the handles' displacement -acting in turn on the incremental deflection of the device's distal end- especially with actuators as those depicted in Figures 10 to 16.

Preferably, handles **400** and **500** are designed to impede or at least limiting as much as possible their relative rotation. Advantageously, handles **400** and **500** are longitudinally constrained by e.g. a guiding mechanism having grooves and guided pins, or via matching slots designed on the inner surface of their lumens.

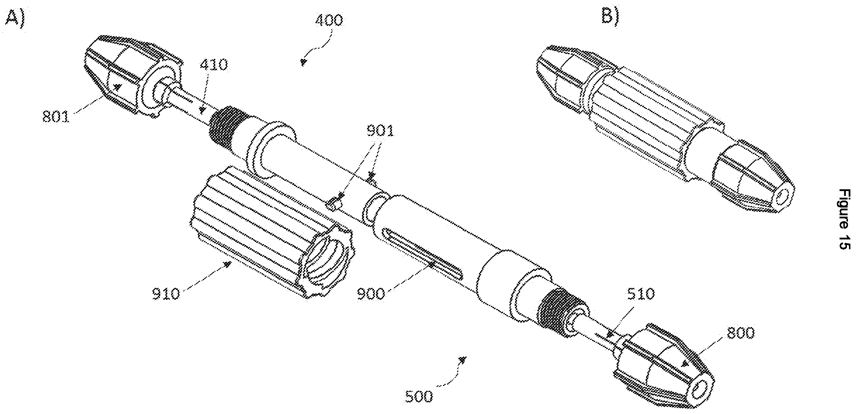

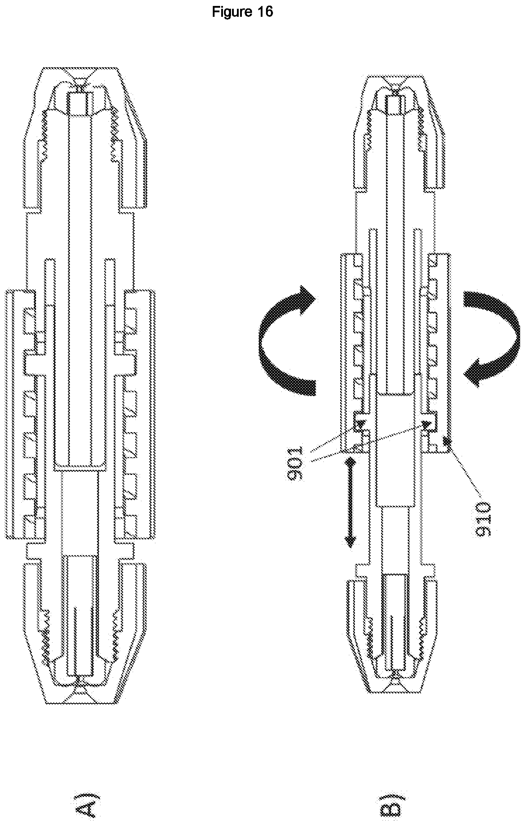

In still another embodiment, one of the first or second handle **400** or **500** comprises a longitudinal slot **900** along its body, and the other of the first or second handle **400** or **500** comprises a protrusion **901** adapted to fit and slide along said longitudinal slot **900.** In this context, the system further comprises a gear rotational actuator **910** adapted to engage said protrusion **901** and operate, upon rotation, a longitudinal relative displacement of said first and second handles **400** and **500** (Figures 15 and 16).

As a way of example, one method (not claimed) of use involves performing a
percutaneous or cutdown procedure to gain access to structures such as, but not limited to, the vasculature, either a vein, an artery, a body lumen or duct, a hollow organ, musculature, fascia, cutaneous tissue, the abdominal cavity, the thoracic cavity, and the like. An introducer, which is usually a hollow, large diameter, hypodermic needle, and the steerable guidewire are placed within the vasculature and the steerable guidewire is advanced through the central lumen of said introducer to be routed proximate to the target treatment site. The introducer can be removed at this time or substantially at the time the guidewire is introduced into the body lumen.

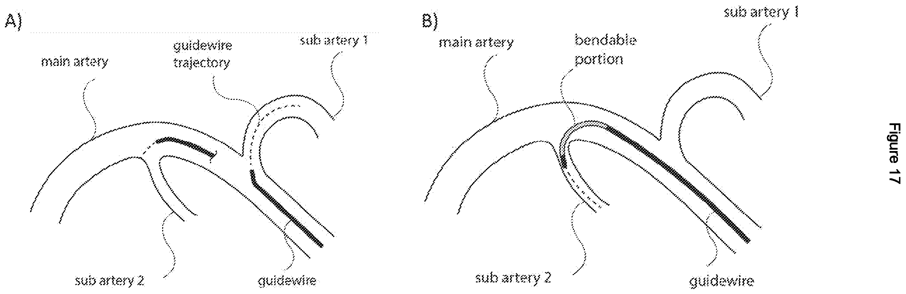

The torque device **300** is assembled or otherwise arranged so that the steerable guidewire **1** is fed within the unitary channel formed by channels **404** and **504,** collet clamps' bores **410** and **510** and, whenever present, hollow passages **806** and **807** of the fasteners **800** and **801,** as well as through the apertures of the foregoing. Deflection of the distal end **102** to varying degrees of curvature, under control from the proximal end **100** or body **101** of the guidewire can be performed. The curve can be oriented along the direction of a branching vessel or vessel curve so that the steerable guidewire can then be advanced into the vessel by way of its high column strength and torqueability (Figure 17).

For instance, when a branch or bend in the body vessel is reached, the first and second handles **400** and **500** are rotated and threaded into the second ends **804** and **805** on an interior of the fasteners **800** and **801** (or vice versa), so that the handles' distal ends **403** and **503** are advanced into the narrowing passage of the fasteners **800** and **801** towards the tapered portions thereof **802, 803.** As distal ends **403, 503** and first ends **802, 803,** respectively, converge, the flanges of spring collet clamps **410** and **510** are compressed around the guidewire to produce sufficient frictional force to grasp the guidewire, thereby locking it relative to the torque device **300.** In this configuration, the torque device **300** is therefore in a closed position and advancement of the guidewire through it is impeded. The user can exploit the torque device **300** as a handle to easily manipulate the guidewire (e.g. twisting and/or laterally move it).

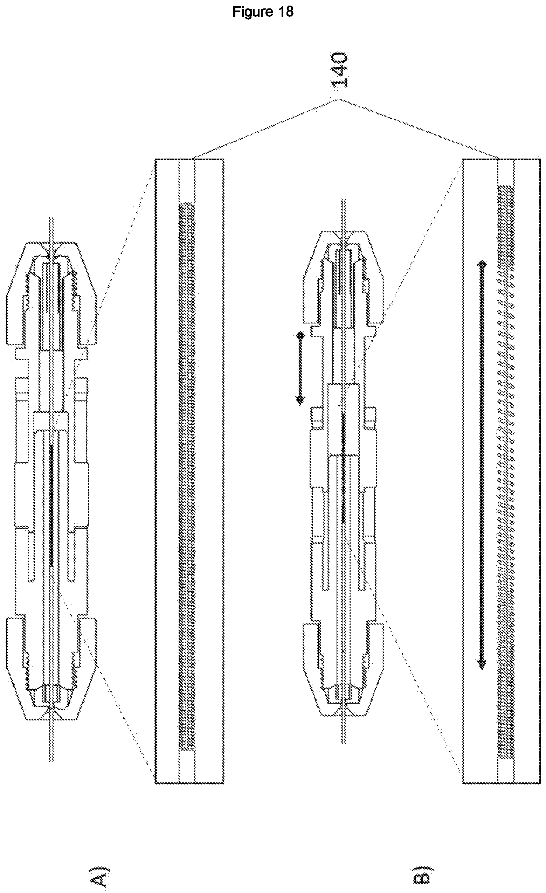

At this point, a relative displacement between first and second handles **400** and **500** can be performed by the user (Figure 18). A connection element between said first and second handles **400** and **500,** such as an accordion-like, corrugated element **600** (Figure 10), a spring clip **700** (Figure 11A and B) or longitudinal slot **900**/protrusion **901** coupled by a gear rotational actuator **910** (Figures 15 and 16), is operated by the user to move away first and second handles **400** and **500** along their common longitudinal axis. Being the torque device **300** anchored on the proximal end **100** or the body **101** of the guidewire on two anchor points **141** and **142** via its first ends **802** and **803,** the displacement of the handles on the designated actuation region **140** imparts a distally-directed force on the bendable portion **104,** that in turn collapses the hollow spaces **103'** in the stress relief portion **120** of the guidewire's bendable region **104,** bringing the stress relief elements **103** closer together and permitting the device's bending towards the target vessel or capillary. The surgeon can also release the stress tension when convenient. To do so, the first and second handles **400** and **500** are moved back in the initial position by acting on the connection element (e.g., spring clip **700** or gear rotational actuator **910**). The fasteners **800** and **801** can be, at this point, unscrewed from distal ends **403** and **503** to allow the guidewire to be fed through the torquer **300.** Once the guidewire is in the desired location and advancement through the body vessel is complete, the torque device **300** may be opened and slidably removed from the guidewire by its proximal end. Once the guidewire is in place, the surgeon can further proceed with the endovascular procedure.

## Claims

1. A steerable guidewire having an elongated body (101) defining a longitudinally-arranged lumen ((200)), said guidewire comprising:
a) a proximal end portion (100);
b) a distal end portion (102) comprising a bendable portion (104) and a tip (105), said bendable portion (104) comprising a reinforcement structure (107) located on a lateral side and a flexible, stress relief portion (120) located on the opposed lateral side, wherein said body (101) is substantially straight from its proximal end portion (100) to its distal end portion (102);
c) a pull wire (106) connected to said bendable portion (104) and extending therefrom up to said proximal end portion (100) along said lumen (200)
d) an actuation region (140) adapted to impart a tension force on the pull wire (106) resulting in a distally-directed force to the bendable region (104) so that the generation of a compression stress will induce a bending which is opposed to the side of the reinforcement structure (107), wherein the actuation region (140) is elastic in nature,
**characterized in that**
i) said pull wire (106) is affixed to said proximal end portion (100) and
ii) the actuation region (140) is located along the proximal end portion (100) and/or the elongated body (101).

2. The guidewire of claim 1, **characterized in that** said actuation region (140) comprises a coiled member.

3. The guidewire of any previous claim, **characterized in that** the reinforcement structure (107) is integral part of the elongated body.

4. The guidewire of any previous claim, **characterized in that** the bendable portion (104) comprises a coiled member.

5. The guidewire of claims 1 to 3, **characterized in that** the flexible, stress relief portion (120) comprises a plurality of cut-outs positioned on one lateral side of the bendable portion (104).

6. A system comprising the device of claims 1 to 5 and an actuator (300) adapted to impart a tension force on the pull wire (106), by acting on the actuation region (140), resulting in a distally-directed force to the bendable region (104).

7. The system of claim 6, **characterized in that** the actuator (300) is a torque device comprising:
a) a first handle (400) comprising:
- an elongated body (401), a proximal end (402) and a distal end (403), said handle (400) defining a lumen (404) along its entire length, and said distal end (403) comprising means (410) for gripping and ungripping an actuation region (140) of the steerable guidewire according to claims 1 to 5;
b) a second handle (500) comprising:
- an elongated body (501), a proximal end (502) and a distal end (503), said handle defining a lumen (504) along its entire length, and said distal end (503) comprising means (510) for gripping and ungripping an actuation region (140) of the steerable guidewire according to claims 1 to 5;
wherein the first and second handles (400) and (500) are arranged in a coaxial configuration so that they can perform a longitudinal relative displacement.

8. The system of claim 7, **characterized in that** said gripping and ungripping means (410) and/or (510) comprise a spring collet clamp.

9. The system of claims 7 or 8, **characterized in that** said distal ends (403) and/or (503) of said first and second handles (400) and (500) comprise a tapered tip adapted to engage said gripping and ungripping means (410) and/or (510) and tighten them upon a longitudinal relative displacement of said first and second handles (400) and (500).

10. The system of claims 7 to 9, **characterized in that** the first and second handles (400) and (500) are connected through an accordion-like, corrugated element 600.

11. The system of claims 7 to 9, **characterized in that** the first and second handles (400) and (500) are connected through a spring clip (700).

12. The system of claims 7 to 9, **characterized in that** the first and second handles (400) and (500) are arranged in a coaxial male-female configuration.

13. The system of claims 7 to 12, **characterized in that** it further comprises a first and second tapered fasteners (800) and (801) adapted to releasably engage and tighten said gripping and ungripping means (410) and (510) of said first and second handles (400) and (500), respectively.

14. The system of claims 12 or 13, **characterized in that** one of the first or second handle (400) or (500) comprises a longitudinal slot (900) along its body, and the other of the first or second handle (400) or (500) comprises a protrusion (901) adapted to fit and slide along said longitudinal slot (900), and comprising a gear rotational actuator (910) adapted to engage said protrusion (901) and operate upon rotation a longitudinal relative displacement of said first and second handles (400) and (500).

## Patentansprüche

1. Ein lenkbarer Führungsdraht mit einem länglichen Körper (101), der ein in Längsrichtung angeordnetes Lumen ((200)) definiert, wobei der Führungsdraht umfasst:
a) einen proximalen Endabschnitt (100);
b) einen distalen Endabschnitt (102), der einen biegbaren Abschnitt (104) und eine Spitze (105) umfasst, wobei der biegbare Abschnitt (104) eine Verstärkungsstruktur (107), die sich auf einer lateralen Seite befindet, und einen flexiblen Spannungsentlastungsabschnitt (120), der sich auf der gegenüberliegenden lateralen Seite befindet, umfasst, wobei der Körper (101) von seinem proximalen Endabschnitt (100) zu seinem distalen Endabschnitt (102) im Wesentlichen gerade ist;
c) einen Zugdraht (106), der mit dem biegbaren Abschnitt (104) verbunden ist und sich von diesem bis zu dem proximalen Endabschnitt (100) entlang des Lumens (200) erstreckt
d) einen Betätigungsbereich (140), der geeignet ist, eine Zugkraft auf den Zugdraht (106) auszuüben, die zu einer distal gerichteten Kraft auf den biegbaren Bereich (104) führt, so dass die Erzeugung einer Druckspannung eine Biegung induziert, die der Seite der Verstärkungsstruktur (107) entgegengesetzt ist, wobei der Betätigungsbereich (140) elastischer Natur ist,
**dadurch gekennzeichnet, dass**
i) der Zugdraht (106) an dem proximalen Endabschnitt (100) befestigt ist und
ii) der Betätigungsbereich (140) befindet sich entlang des proximalen Endabschnitts (100) und/oder des länglichen Körpers (101).

2. Führungsdraht nach Anspruch 1, **dadurch gekennzeichnet, dass** der Betätigungsbereich (140) ein spiralförmiges Element umfasst.

3. Führungsdraht nach einem der vorhergehenden Ansprüche, **dadurch gekennzeichnet, dass** die Verstärkungsstruktur (107) integraler Bestandteil des länglichen Körpers ist.

4. Führungsdraht nach einem der vorhergehenden Ansprüche, **dadurch gekennzeichnet, dass** der biegbare Abschnitt (104) ein spiralförmiges Element umfasst.

5. Führungsdraht nach einem der Ansprüche 1 bis 3, **dadurch gekennzeichnet, dass** der flexible Spannungsentlastungsabschnitt (120) eine Vielzahl von Aussparungen aufweist, die auf einer Seite des biegbaren Abschnitts (104) angeordnet sind.

6. System mit der Vorrichtung nach einem der Ansprüche 1 bis 5 und einem Aktuator (300), der so beschaffen ist, dass er eine Zugkraft auf den Zugdraht (106) ausübt, indem er auf den Betätigungsbereich (140) einwirkt, was zu einer distal gerichteten Kraft auf den biegbaren Bereich (104) führt.

7. System nach Anspruch 6, **dadurch gekennzeichnet, dass** der Aktuator (300) eine Drehmomentvorrichtung ist, die Folgendes umfasst:
a) einen ersten Griff (400), der Folgendes umfasst:
- einen länglichen Körper (401), ein proximales Ende (402) und ein distales Ende (403), wobei der Griff (400) ein Lumen (404) entlang seiner gesamten Länge definiert und das distale Ende (403) Mittel (410) zum Greifen und Lösen eines Betätigungsbereichs (140) des lenkbaren Führungsdrahtes nach den Ansprüchen 1 bis 5 umfasst;
b) einen zweiten Griff (500), der Folgendes umfasst:
- einen länglichen Körper (501), ein proximales Ende (502) und ein distales Ende (503), wobei der Griff ein Lumen (504) entlang seiner gesamten Länge definiert und das distale Ende (503) Mittel (510) zum Greifen und Lösen eines Betätigungsbereichs (140) des lenkbaren Führungsdrahtes nach den Ansprüchen 1 bis 5 umfasst;
wobei die ersten und zweiten Griffe (400) und (500) in einer koaxialen Konfiguration angeordnet sind, so dass sie eine relative Längsverschiebung durchführen können.

8. System nach Anspruch 7, **dadurch gekennzeichnet, dass** die Greif- und Lösemittel (410) und/oder (510) eine Feder-Spannzangenklemme umfassen.

9. System nach Anspruch 7 oder 8, **dadurch gekennzeichnet, dass** die distalen Enden (403) und/oder (503) der ersten und zweiten Griffe (400) und (500) eine konische Spitze aufweisen, die geeignet ist, mit den Greif- und Lösemitteln (410) und/oder (510) in Eingriff zu kommen und sie bei einer relativen Längsverschiebung der ersten und zweiten Griffe (400) und (500) festzuziehen.

10. System nach einem der Ansprüche 7 bis 9, **dadurch gekennzeichnet, dass** der erste und zweite Griff (400) und (500) durch ein akkordeonartiges, gewelltes Element 600 verbunden sind.

11. System nach einem der Ansprüche 7 bis 9, **dadurch gekennzeichnet, dass** der erste und zweite Griff (400) und (500) durch eine Federklammer (700) verbunden sind.

12. System nach einem der Ansprüche 7 bis 9, **dadurch gekennzeichnet, dass** die ersten und zweiten Griffe (400) und (500) in einer koaxialen Steckverbindungskonfiguration angeordnet sind.

13. System nach den Ansprüchen 7 bis 12, **dadurch gekennzeichnet, dass** es ferner einen ersten und einen zweiten konischen Befestiger (800) und (801) umfasst, die so beschaffen sind, dass sie mit den Greif- und Lösemitteln (410) und (510) des ersten bzw. zweiten Griffs (400) und (500) lösbar in Eingriff kommen und diese festziehen.

14. System nach Anspruch 12 oder 13, **dadurch gekennzeichnet, dass** einer der ersten oder zweiten Griffe (400) oder (500) einen Längsschlitz (900) entlang seines Körpers aufweist und der andere der ersten oder zweiten Griffe (400) oder (500) einen Vorsprung (901) aufweist, der so angepasst ist, dass er entlang des Längsschlitzes (900) passt und gleitet, und einen Zahnrad-Drehaktuator (910) aufweist, der so angepasst ist, dass er in den Vorsprung (901) eingreift und bei Drehung eine relative Längsverschiebung der ersten und zweiten Griffe (400) und (500) bewirkt.

## Revendications

1. Un guide souple et directionnel ayant un corps allongé (101) définissant une lumière disposée longitudinalement ((200)), ledit guide souple et directionnel comprenant :
a) une portion proximale (100) ;
b) une portion d'extrémité distale (102) comprenant une portion pliable (104) et une pointe (105), ladite portion pliable (104) comprenant une structure de renforcement (107) située sur un côté latéral et une portion flexible de décharge de contrainte (120) située sur le côté latéral opposé, dans laquelle ledit corps (101) est sensiblement droit de sa portion d'extrémité proximale (100) à sa portion d'extrémité distale (102)
c) un fil de traction (106) relié à ladite portion flexible (104) et s'étendant à partir de celle-ci jusqu'à ladite portion proximale (100) le long de ladite lumière (200).
d) une région d'actionnement (140) adaptée pour transmettre une force de tension sur le fil de traction (106) résultant en une force dirigée distalement vers la région pliable (104) de sorte que la génération d'une contrainte de compression induira une flexion opposée au côté de la structure de renforcement (107), la région d'actionnement (140) étant de nature élastique,
**caractérisé par le fait que**
i) ledit fil de traction (106) est fixé à ladite portion proximale (100) et
ii) la région d'actionnement (140) est située le long de la portion proximale (100) et/ou du corps allongé (101).

2. Le fil-guide de la revendication 1, **caractérisé en ce que** ladite région d'actionnement (140) comprend un élément enroulé.

3. Fil-guide de toute revendication précédente, **caractérisé en ce que** la structure de renforcement (107) fait partie intégrante du corps allongé.

4. Fil-guide de toute revendication précédente, **caractérisé en ce que** la portion flexible (104) comprend un élément enroulé.

5. Le fil-guide des revendications 1 à 3, **caractérisé en ce que** la portion flexible de décharge de contrainte (120) comprend une pluralité de découpes positionnées sur un côté latéral de la portion flexible (104).

6. Système comprenant le dispositif des revendications 1 à 5 et un actionneur (300) adapté pour transmettre une force de tension au fil de traction (106), en agissant sur la région d'actionnement (140), ce qui entraîne une force dirigée distalement vers la région pliable (104).

7. Le système de la revendication 6, **caractérisé en ce que** l'actionneur (300) est un dispositif de couple comprenant :
a) une première poignée (400) comprenant :
- un corps allongé (401), une extrémité proximale (402) et une extrémité distale (403), ladite poignée (400) définissant une lumière (404) sur toute sa longueur, et ladite extrémité distale (403) comprenant des moyens (410) de préhension et de libération d'une région d'actionnement (140) du guide souple et directionnel selon les revendications 1 à 5 ;
b) une seconde poignée (500) comprenant :
- un corps allongé (501), une extrémité proximale (502) et une extrémité distale (503), ladite poignée définissant une lumière (504) sur toute sa longueur, et ladite extrémité distale (503) comprenant des moyens (510) de préhension et de libération d'une région d'actionnement (140) du guide souple et directionnel selon les revendications 1 à 5 ;
dans lequel la première et la deuxième poignée (400) et (500) sont disposées dans une configuration coaxiale de sorte qu'elles peuvent effectuer un déplacement relatif longitudinal.

8. Le système de la revendication 7, **caractérisé en ce que** lesdits moyens de préhension et de libération (410) et/ou (510) comprennent une pince à ressort.

9. Le système des revendications 7 ou 8, **caractérisé en ce que** lesdites extrémités distales (403) et/ou (503) desdites première et deuxième poignées (400) et (500) comprennent une pointe effilée adaptée pour engager lesdits moyens de préhension et de libération (410) et/ou (510) et les serrer lors d'un déplacement relatif longitudinal desdites première et deuxième poignées (400) et (500).

10. Le système des revendications 7 à 9, **caractérisé en ce que** la première et la deuxième poignée (400) et (500) sont reliées par un élément ondulé en forme d'accordéon 600.

11. Le système des revendications 7 à 9, **caractérisé en ce que** la première et la deuxième poignée (400) et (500) sont reliées par un clip à ressort (700).

12. Le système des revendications 7 à 9, **caractérisé en ce que** la première et la deuxième poignée (400) et (500) sont disposées selon une configuration coaxiale mâle-femelle.

13. Le système des revendications 7 à 12, **caractérisé en ce qu'**il comprend en outre une première et une deuxième attaches coniques (800) et (801) adaptées pour engager et serrer de manière libérable lesdits moyens de préhension et de libération (410) et (510) desdites première et deuxième poignées (400) et (500), respectivement.

14. Le système des revendications 12 ou 13, **caractérisé en ce que** l'une de la première ou de la deuxième poignée (400) ou (500) comprend une fente longitudinale (900) le long de son corps, et l'autre de la première ou de la deuxième poignée (400) ou (500) comprend une proéminence (901) adaptée pour s'adapter et glisser le long de ladite fente longitudinale (900), et comprenant un actionneur rotatif à engrenage (910) adapté pour engager ladite proéminence (901) et opérer lors de la rotation un déplacement relatif longitudinal desdites première et deuxième poignées (400) et (500).
